# EUROPEAN PATENT APPLICATION

(11) **EP 2 216 015 A1**
(43) Date of publication of application: **11.08.2010**
(21) Application number: 08844909.5
(22) Date of filing: 29.10.2008
(51) Int. Cl.: A61K 9/10, A61K 9/107, A61K 9/14

(54) **POORLY SOLUBLE SUBSTANCE-SURFACTANT COMPLEX PRODUCT, AND PROCESS FOR PRODUCTION THEREOF**

(30) Priority: 02.11.2007 JP 2007286302
(71) Applicant: Aspion Co., Ltd., Hyogo 650-0047 (JP)
(72) Inventor: FUJII, Takeru, Kobe-shi Hyogo 650-0047 (JP); HAYAKAWA, Eiji, Kobe-shi Hyogo 650-0047 (JP); HIRATA, Akihiko, Kobe-shi Hyogo 650-0047 (JP)
(74) Representative: Denison, Christopher Marcus
(86) International application number: PCT/JP2008/070149
(87) International publication number: WO 2009/057808

(57) **Abstract**

This invention is intended to improve the solubility and permeability of low-solubility drugs, including drugs hardly soluble in water, classified as Class 2 or 4 in accordance with BCS by modifying such drugs into S/W, S/O, or S/O/W preparations. The S/W, S/O, or S/O/W preparations of low-solubility drugs of this invention are prepared by a method for preparing a composite of a low- solubility drug and surfactant by introducing air or nonflammable gas into the gas phase in the upper portion of a liquid level of the dispersion, dissolution, and emulsification tanks, respectively, at a pressure of 1 to 10 atm.

## Description

### Technical Field

The present invention relates to improvement in low solubility (LS) and low permeability of drugs indicated in the Biopharmaceutical Classification System (BCS). Also, the invention relates to a pharmaceutical preparation containing a composite of a low-solubility drug and surfactant with enhanced solubility and improved permeability due to enclosure of low- solubility drugs with surfactant and a process for production thereof.

### Background Art

Since combinatorial chemistry (CC) or high throughput screening (HTS) was developed, the synthesis of numerous compounds or the evaluation/screening of many candidate compounds became feasible. At the same time, many candidate compounds for new drugs that are to be screened for are low- solubility compounds that are not dissolved in water and have very low solubility. This is a serious issue of concern in the field of drug development (Folia Pharmacol. Jpn. (Nippon Yakurigaku Zasshi) 127, 213-216, 2006).

Gordon Amidon suggested the Biopharmaceutical Classification System (BCS) as a method for describing oral absorption by using compound solubility in combination with membrane permeability. At present, such system is employed as a method for evaluating bioequivalence (BE) between (or among) pharmaceutical preparations.

BCS is defined in accordance with the following guidance of the U.S. Food and Drug Administration (USFDA).

Guidance for industry: Waiver of *in vivo* bioavailability and bioequivalence studies for immediate-release solid oral dosage forms based on a biopharmaceutics classification system

BCS divides drug substances into four classes: Class 1 (HS, HP): high solubility (HS) and high permeability (HP); Class 2 (LS, HP): low solubility (LS) and high permeability (HP); Class 3 (HS, LP): high solubility (HS) and low permeability (LP); and Class 4 (LS, LP): low solubility (LS) and low permeability (LP), based on solubility in 250 ml of buffer over the pH range of 1.0 to 7.5 in combination with membrane permeability, such as permeability of cell membranes of, for example, Caco-2 or MDCK cells derived from epithelial cells of the digestive tract or permeability of artificial membranes.

Meanwhile, Goto et al. demonstrated drug preparations that were prepared by enclosing hydrophilic anticancer drugs using highly lipophilic surfactant as novel emulsion carriers by stabilizing and reducing the size of drug substances, so that the resultants can be used for injection preparations, removing moisture from the internal aqueous phase to prepare Solid-in-Oil (S/O) preparations and Solid-in-Oil-in-Water emulsions (S/O/W) preparations in which S/O preparations are further dissolved in water (Proceedings of the 36th SPG Forum, 50 to 53, 2001).

Further, they prepared S/O/W preparations enclosing insulin (i.e., a biologically active protein), administered the prepared preparations orally to diabetic rat models, and demonstrated that such preparations would lower blood glucose levels (International Journal of Pharmaceutics 252; 271-274, 2003). Furthermore, they demonstrated that oral absorption and even percutaneous absorption of antiinflammatory drugs, would be enhanced (JP Patent Publication (kokai) No. 2004-43355 A, WO 2005/094789, and WO 2006/025583).

S/O or S/O/W preparations are **characterized in that** such preparations equalize molecular properties, regardless of whether the target substances are proteins or chemical compounds ranging from drugs to food or cosmetic products. For example, HS in accordance with BCS (i.e., drugs readily soluble in water) are enclosed with highly lipophilic surfactant to modify hydrophilic drugs into lipophilic one, and the resultant is dispersed in lipophilic bases.

Accordingly, the aforementioned techniques were intended to prepare drugs targeted by S/O or S/O/W preparations and classified as Class 3 in accordance with BCS in the form of S/O preparations, so as to modify the drugs of Class 3 into those of Class 1.

In the past, there was an example of preparation of S/O by dissolving solid powder of water-soluble drugs in a solvent using surfactant to prepare a solid dispersion (JP Patent Publication (kokai) No. 2004-8837 A); however, the drugs used therefor were of Class 3, which was of high solubility, and the targets thereof were not drugs that were lowly soluble in water (LS) classified as Class 2 or 4.

### Disclosure of the Invention

Up to the present, there has not been any adequate means for enhancing the solubility or permeability of candidate compounds or drugs that are classified as Class 2 or 4 in accordance with BCS. Thus, provision of an adequate means for enhancing solubility or permeability of candidate compounds or drugs that are classified as Class 2 or 4 can be said to be a major issue in drug development (Table 1).

**Table 1**

| Biopharmaceutical Classification System(BCS) | |
|---|---|
| Class 1 | Class 2 |
| HS-HP | LS-HP |
| High Solubility | Low Solubility |
| High Permeability | High Permeability |

| Class 3 | Class 4 |
|---|---|
| HS-LP | LS-LP |
| High Solubility | Low Solubility |
| Low Permeability | Low Permeability |

When micelle preparations such as liposomes or emulsions are prepared in order to enhance solubility or permeability of low-solubility drugs, for example, heating is effective, as well as pH adjustment or addition of salts, in order to enhance solubility of drugs at the time of drug preparation. Because of the difference in boiling points of water and an organic solvent, however, mixing with heating could cause bumping or serious explosion, disadvantageously. When thermal sterilization of preparations is intended, further, the preparations may be thermally expanded and destroyed because of the properties of the internal aqueous or oil phases.

Accordingly, the problem with the preparation of low-solubility drugs to result in micelle preparations is also applicable to S/O or S/O/W preparations, and the way in which low-solubility drugs are solubilized would be an issue of concern.

Further, resolving and overcoming problems in production of S/O or S/O/W preparations is required to establish S/O or S/O/W preparations as a technique of modifying low- solubility drugs that are classified as Class 2 or Class 4 in accordance with BCS into those of Class 1 in drug development.

Formation of S/O or S/O/W micelle preparations is a method that is excellent in terms of stability and absorption. Since it is characterized by the use of hydrophilic drugs having a solid phase as the innermost phase, such micelles are of Class 3 of high solubility (HS) and low permeability (LP) in accordance with BCS, although they are not of Class 2 or Class 4. Therefore, the present invention is intended to modify S/O or S/O/W micelle preparations in order that they may be classified as Class 2 or Class 4.

In order to improve water solubility and permeability of low- solublility drugs classified as Class 2 or Class 4 in accordance with BCS (HS, HP), the present inventors improved a method for producing S/O/W preparations using S/O preparations and succeeded in improving the solubility of low- solublility drugs in water. Further, they prepared a composite comprising low- solublility drugs enclosed by highly hydrophilic surfactant, dispersed the resultant in that state in water, and then succeeded in preparing solid-in-water (S/W) preparations.

In order to resolve the above problems, the present invention also provides a pharmaceutical preparation comprising a composite of a low- solublility drug and surfactant that comprises low- solublility drugs enclosed by a molecular associate of surfactant and a method for producing thereof. The present invention is described in detail below.

The term "low solubility drugs" used herein refers to drugs exhibiting a degree of solubility that is equal to or less than the maximal drug content in 1 unit of the relevant preparation in 250 ml of buffer with a pH level of 1.0 to 7.5, and such drugs are classified as "low-solubility (LS)" in accordance with the Biopharmaceutical Classification System (BCS). Such low solubility drugs are classified as Class 2 or 4 according to BCS.

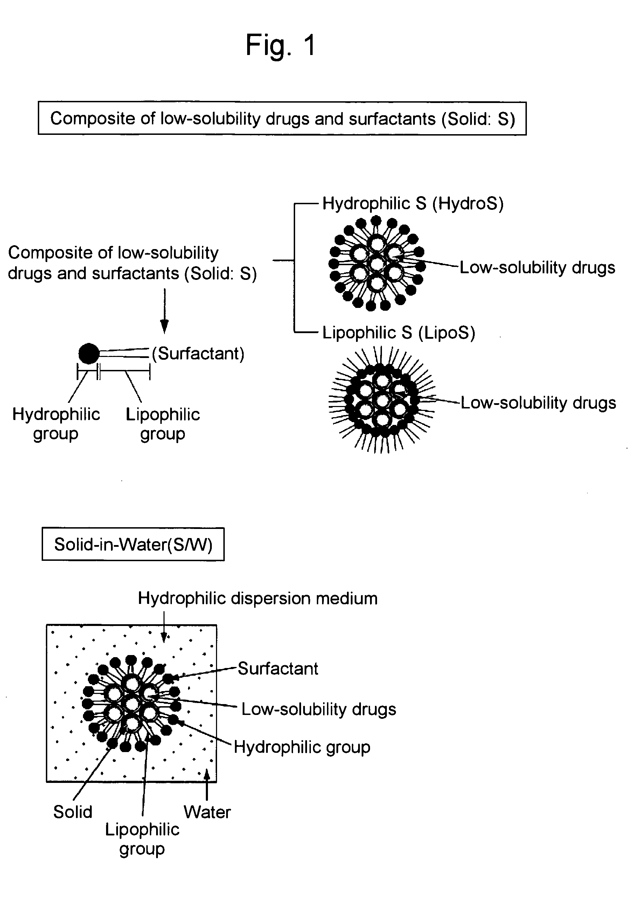

A preferable embodiment is an S/W (solid-in-water) preparation comprising a composite of a low-solubility drug and surfactant dispersed in water as shown in Fig. 1.

Composites of low- solublility drugs and surfactant comprising low-solubility drugs enclosed by a molecular associate of surfactant can be divided into two types depending on whether low-solublility drugs are lipophilic or hydrophilic.

When low-solubility drugs are lipophilic, surfactant enclose low-solubility with lipophilic groups thereof facing inward and hydrophilic groups thereof facing outward, and lipophilic low-solubility are modified into hydrophilic low-solubility drugs. Hereafter, the abbreviation "HydroS" is used to refer to such drugs. When preparation of S/W preparations is intended, HydroS is dispersed in water.

When low-solubility drugs are hydrophilic, surfactant enclose low-solubility drugs with hydrophilic groups thereof facing inward and lipophilic groups thereof facing outward, and hydrophilic low-solubility drugs are modified into lipophilic low-solubility drugs. Hereafter, the abbreviation "LipoS" is used to refer to such drugs. When preparation of S/O preparations is intended, LipoS is dispersed in oil.

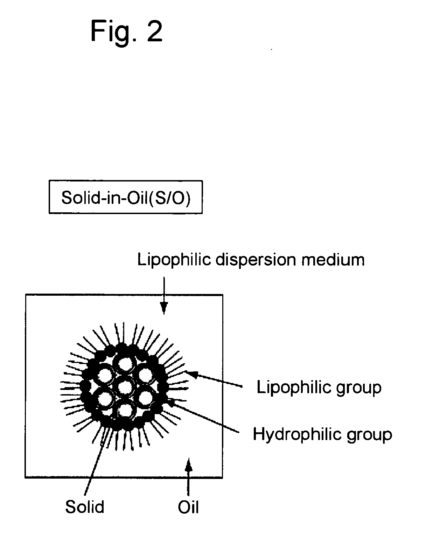

Fig. 2 shows an S/O (solid-in-oil) preparation comprising a composite of a low-solubility drug and surfactant dispersed in oil.

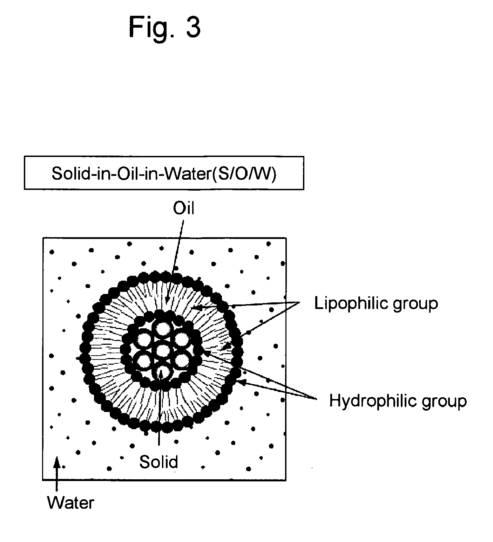

Further, a dispersion of an S/O in water is an S/O/W (solid-in-oil-in-water) (shown in Fig. 3).

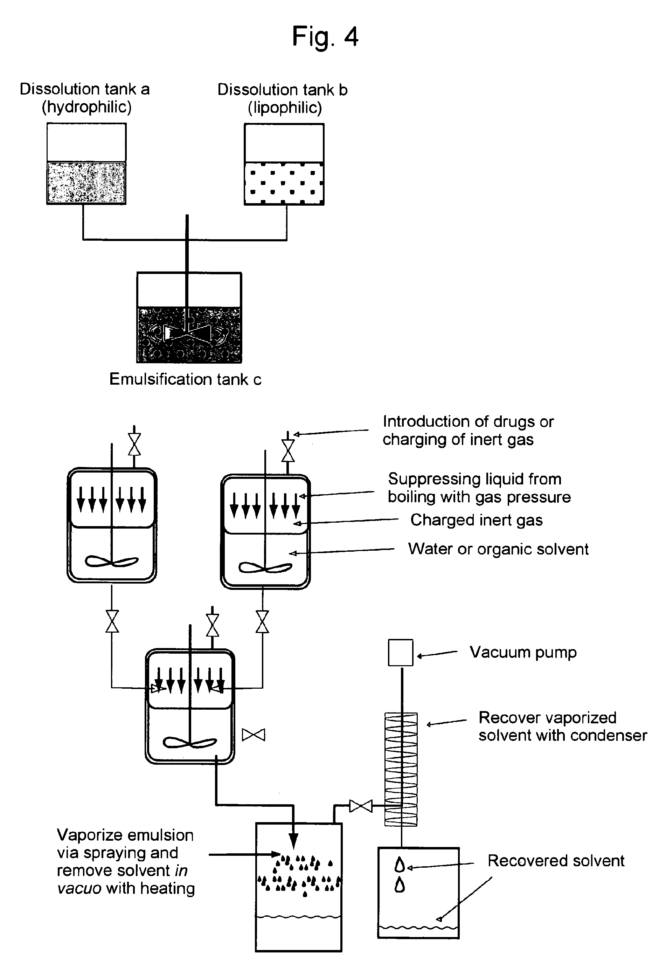

Fig. 4 shows a process of producing S/W, S/O, and S/O/W.

S/O and/or S/O/W can be produced by a method comprising a step of emulsifying or dispersing low-solubility drugs in a solvent using surfactant, a step of removing a solvent or moisture from the resulting emulsion or dispersion to prepare a composite product of a low-solubility drug and surfactant , and a step of dispersing the composite product of a low-solubility drug -surfactant in at least 1 type of oil selected from the group consisting of a vegetable oil and a synthetic oil or fat.

All steps are characterized by production of a composite of a low-solubility drug and surfactant (hereafter HydroS and LipoS may be referred to as "solid" or "S").

A composite of a low-solubility drug and surfactant (S) can be produced by a method comprising a step of emulsifying and/or dispersing low-solubility drugs in a solvent using surfactant and a step of removing a solvent or moisture from the resulting emulsion and/or dispersion.

More specifically, S/O can be produced by a method comprising:
(A) a step of dissolving and/or dispersing low-solubility drugs in an aqueous solution to obtain a solution and/or dispersion;
(B) a step of liquefying and dissolving a lipophilic surfactant via heating or other means or dissolving and/or dispersing a lipophilic surfactant in an organic solvent to obtain a solution and/or dispersion;
(C) a step of mixing the solution and/or dispersion obtained in step (A) with the solution and/or dispersion obtained in step (B) to obtain an emulsion or dispersion;
(D) a step of removing a solvent or moisture from the emulsion or dispersion obtained in step (C); and
(E) a step of dispersing the composite product of a low-solubility drug and surfactant obtained in step (D) in at least 1 type of oil selected from the group consisting of a vegetable oil and a synthetic oil or fat.

Step (A) and step (B) may be carried out in any order, and these steps may be carried out simultaneously.

In step (A), low-solubility drugs are dissolved and dispersed in an aqueous solution via various methods for enhancing solubility. Examples of such methods include a method involving the use of a pH regulator to adjust pH to a level that yields high solubility, addition of a solubilizer, and a method involving heating or heating and pressurization with an inert gas.

In the above-described method, further, low-solubility drugs may be added in the form of fine powder in step (B) to obtain a solution or dispersion. Alternatively, water may further be added thereto to obtain an emulsion. An example of a means for liquefaction of a lipophilic surfactant via heating or other means in step (B) is a method involving heating or heating and pressurization with an inert gas.

Also, S/O/W can be produced by a method comprising:
(F): a step of dispersing S/O obtained in step (E) in purified water or regulated water, such as buffer.

S/W can be produced by a method comprising:
(i) a step of dissolving and/or dispersing low-solubility drugs in an organic solvent to obtain a solution and/or dispersion;
(ii) a step of liquefying and dissolving a hydrophilic surfactant via heating or other means or dissolving and/or dispersing a hydrophilic surfactant in water to obtain a solution and/or dispersion;
(iii) a step of adding the solution and/or dispersion in an organic solvent obtained in step (i) to the solution and/or dispersion obtained in step (ii) to obtain an emulsion or dispersion;
(iv) a step of removing a solvent or moisture from the emulsion or dispersion obtained in step (iii) to obtain S; and
(v) a step of dispersing S obtained in step (iv) in purified water or regulated water, such as buffer. Step (i) and step (ii) may be carried out in any order, and these steps may be carried out simultaneously.

In step (ii), low-solubility drugs may be dissolved and dispersed in an aqueous solution in the form of fine powder to obtain a solution and/or dispersion. Alternatively, an organic solvent may further be added thereto to obtain an emulsion. An example of a means for liquefaction of a hydrophilic surfactant via heating or other means in step (ii) is a method involving heating or heating and pressurization with an inert gas.

Preferably, steps (A), (B), (C), and at least any one of steps (i), (ii), and (iii) are carried out by introducing air or nonflammable gas therein. Preferably, pressure of the air or inert gas to be introduced is between 1 and 10 atm. More preferably, air or nonflammable gas is introduced into a gas phase in the upper portion of the liquid level at the time of dissolution, dispersion, or emulsification in steps (B), (C), (i), and (iii). Preferably, pressure of the air or inert gas to be introduced is between 1 and 10 atm.

The nonflammable gas can be at least one type of gas selected from the group consisting of nitrogen, carbonic acid, helium, and argon gases.

Removal of a solvent and/or moisture of step (D) or (iv) can be carried out by means of, for example, vacuum-freeze drying, drying under reduced pressure, microwave drying by bulking method, freeze-drying and grinding, or spray drying. This step can produce a composite product of a low-solubility drug and surfactant (S) enclosing low-solubility drugs in a solid state. According to the present invention, a composite in which the low-solubility drugs have been subjected to removal of a solvent and/or moisture via such means is defined as "enclosing low-solubility drugs in a solid state."

The surfactant are classified as anionic, cationic, amphoteric, and nonionic surfactant. Surfactant comprise in their molecules hydrophilic groups and lipophilic groups, and surfactant are classified as hydrophilic or lipophilic surfactant, depending on a ratio of hydrophilic and lipophilic groups (HLB values).

The hydrophilic-lipophilic balance of surfactant is expressed using the hydrophilic lipophilic balance (HLB) values as the indicators. HLB values range from 0 to 20, an HLB value closer to 0 indicates higher lipophilic properties, and an HLB value closer to 20 indicates higher hydrophilic properties.

Hydrophilic surfactant generate O/W emulsions at an HLB value of approximately 8 or higher. Lipophilic surfactant generate W/O emulsions at an HLB value of less than approximately 8.

Lipophilic surfactant are used to generate S/O emulsions. Hydrophilic surfactant in an amount that is half or less of the amount of lipophilic surfactant can be added. The amount is preferably 10% or less.

An S/O/W preparation obtained by dispersing an S/O oil in an aqueous phase is prepared by adding a hydrophilic surfactant to the aqueous phase. In this case, lipophilic or hydrophilic surfactant may also be added to the oil phase in an amount that is half or less of the amount of a hydrophilic surfactant to be added to the aqueous phase. The amount is preferably 10% or less. Also, a lipophilic surfactant may be added to the aqueous phase. The amount of a lipophilic surfactant to be added to the aqueous phase is half or less of the amount of the hydrophilic surfactant, and the amount is preferably 10% or less.

Hydrophilic surfactant are used to generate S/W emulsions. Lipophilic surfactant in an amount that is half or less of the amount of hydrophilic surfactant can be added. The amount is preferably 10% or less.

Examples of hydrophilic surfactant include anionic, cationic, amphoteric, and nonionic surfactant described below.

Examples of anionic surfactant that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, sodium lauryl sulfate.

Examples of cationic surfactant that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactant that can be used include N-alkyl β-aminopropionic acid salt and N-alkyl β-iminodipropionic acid salt.

Examples of water-soluble nonionic surfactant that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among nonionic surfactant, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, and polyglyceryl fatty acid ester are preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil, trade name: Cremophor EL or EL-P, BASF Japan Ltd.) is particularly preferable. As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As polyoxyethylene polyoxypropylene glycol copolymers, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is preferable.

Examples of lipophilic surfactant include: sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.

The S/O, S/O/W, and S/W preparations of the present invention can be in the form of liquid preparations, and liquid preparations can be in the form of oral preparations, preparations for external use, injection preparations, eye drops, nasal drops, pulmonary preparations, suppositories, or cosmetic products.

Also, the S/O, S/O/W, and S/W preparations of the present invention can be in the form of solid preparations. Examples of solid preparations include powders, granules, capsules, and tablets.

This description includes part or all of the contents as disclosed in the description and/or drawings of Japanese Patent Application No. 2007-286302, which is a priority document of the present application.

### Brief Description of the Drawings

Fig. 1 shows a composite of a low-solubility drug and surfactant (Solid: S).
Fig. 2 shows a product comprising a composite of a low-solubility drug and surfactant (Solid-in-Oil (S/O)).
Fig. 3 shows a product comprising a composite of a low-solubility drug and surfactant (Solid-in-Oil-in-Water (S/O/W)).
Fig. 4 shows a product comprising a composite of a low-solubility drug and surfactant.
Fig. 5 shows the particle size distribution of Production Example 2.
Fig. 6 shows photographed images of Production Example 2 and of Control Example 1.
Fig. 7 shows the results of a test of percutaneous administration of Production Example 2 and a solution of ubiquinone in ethanol to rats.
Fig. 8 shows the results of cross-over test of percutaneous administration of Control Example 3 and the DFNa-S/O suspension of Preparation Example 3 (10 mg/ml) to rabbit's auricle. Since the blood dynamics of Preparation Example 3 is substantially the same as that of Control Example 3, the amount of production of the DFNa-S/O suspensions achieved by the method of producing S/O preparations of low-solubility drugs according to the present invention was elevated by 10 times higher than that of conventional techniques.

### Best Modes for Carrying out the Invention

### 1. Introduction

The S/O low-solubility drug preparations of the present invention can be produced with the use of three tanks; i.e., a water tank, an organic solvent tank, and an emulsification tank, for example. Specifically, such drug preparations can be produced in the following manner. Low-solubility drugs are dispersed in a water tank containing an aqueous solution, surfactant are dispersed in an organic solvent tank containing an organic solvent, and the solutions are heated to enhance solubility. Subsequently, these two types of solutions are kept heated, and the solutions are transferred in that state to an emulsification tank, followed by agitation. In order to inhibit bumping or vapor explosion caused at the time of emulsification and to accelerate emulsification, air or nonflammable gas is introduced into a gas phase in the upper portion of the liquid level of each tank, and the pressure at the liquid level is maintained between 1 atm and 10 atm or higher so as to enhance solubility of low-solubility drugs and prepare the drugs in the form of S/O preparations. Thus, preparations comprising low-solubility drugs nanodispersed therein can be produced.

Production of S/O, S/O/W, or S/W preparations of low-solubility drugs of the present invention is divided into four steps: a step of mixing water and an organic solvent to obtain an emulsion; a step of removing a solvent and/or moisture; a step of dispersing low-solubility drugs in oil (S/O) or in water (S/W or S/O/W), and a step of producing preparations.

Any emulsifiers that can be used for pharmaceutical preparations can be used as the surfactant of the present invention without particular limitation. Examples thereof include nonionic surfactant, anionic surfactant, cationic surfactant, amphoteric surfactant, and bile salt.

The organic solvent that is used in the present invention is an organic solvent that can be used for production of pharmaceutical products. Specific examples thereof include methylene chloride, toluene, xylene, acetone, n-hexane, and a mixture of any thereof.

The air that is used in the present invention is external air, any nonflammable gas that can be used for production of pharmaceutical products can be used, and one or more types of gases selected from the group consisting of nitrogen, carbonic acid, helium, and argon gases can be used.

### 2. Low-solubility drugs

The term "low-solubility drugs " used herein refers to drugs exhibiting a degree of solubility that is equal to or less than the maximal drug content in 1 unit of the relevant preparation in 250 ml of buffer with a pH level of 1.0 to 7.5, and such drugs are classified as Class 2 or 4 according to BCS.

Examples of low-solubility drugs include, but are not limited to: antiviral agents such as acyclovir; steroidal anti-inflammatory agents such as fluorometholone, dexamethasone, and prednisolone or non-steroidal anti-inflammatory agents such as indomethacin and diclofenac sodium; hypotensive agents such as griseofulvin, nifedipine, and nicardipine; anticancer agents such as paclitaxel, Adriamycin, docetaxel, daunomycin, methotrexate, mitomycin C, camptothecin, taxol, vincristine, and derivatives of any thereof; macrolide antibiotics such as Ilotycin, erythromycin, and clarithromycin; antifungal agents such as amphotericin B, itraconazole, and miconazole; hormones such as estradiol, testosterone, progesterone, diethylstilbestrol, and derivatives of any thereof; and prostaglandin or prostacycline agents such as Cilostazol. Further, the drugs of the present invention exhibiting the degree of solubility equivalent to that of the above drugs are included without particular limitation. Any drugs with low solubility that are administered to patients ranging from babies to aged persons and difficult to dose thereof include, for example: NSAIDs drugs developing gastrointestinal injuries, a therapeutic agent for increased urinary frequency that metabolizes a drug rapidly, an antiemetic agent that is difficult to dose, an anti-migraine agent, an anti-dementia agent, an antiparkinsonism agent, an antihypertensive agent, an anti-hyperlipidemia agent, an anti-asthma agent, a therapeutic agent for atopic dermatitis, a therapeutic agent for psoriasis, an antirheumatic agent, a therapeutic agent for leukoplakia, and an agent for preventing imminent abortion.

### 3. Method for preparing S

As described above, S (HydroS or LipoS) can be prepared by a method comprising:
(I) a step of dissolving and/or dispersing low-solubility drugs in an aqueous solution or organic solvent to obtain a solution and/or dispersion;
(II) a step of liquefying and dissolving a lipophilic or hydrophilic surfactant via heating or other means or dissolving and/or dispersing a lipophilic or hydrophilic surfactant in an organic solvent to obtain a solution and/or dispersion;
(III) a step of mixing the solution and/or dispersion obtained in step (I) with the solution and/or dispersion obtained in step (II) to obtain an emulsion or dispersion; and
(IV) a step of removing a solvent and/or moisture from the emulsion or dispersion obtained in step (III).

(Step (I) and step (II) may be carried out in any order, and these steps may be carried out simultaneously. In the above-described method, further, low-solubility drugs may be added in the form of fine powder in step (II) to obtain a solution or dispersion. Alternatively, water may further be added thereto to obtain an emulsion.)

Hereafter, such steps are described in greater detail.

### 3-1.

### 3-1-1. Production of LipoS

### Step (I) Dissolution and/or dispersion of low-solubility drugs in aqueous solution

Low-solubility drugs can be dissolved in water with the use of a solubilizer. Examples of solubilizers for low-solubility drugs include water-soluble acids such as citric acid, adipic acid, lactic acid, phosphoric acid, and carbonic acid compounds, water-soluble bases such as sodium hydroxide, ammonium hydroxide, and sodium phosphate compounds, clathrate compounds of amino acids, proteins, urea, and cyclodextrin, various types of nucleic acids, and various types of saccharides. Such solubilizers can be used alone or in combinations of two or more. Heating and/or pressurization can also be carried out, in order to efficiently dissolve or disperse low-solubility drugs in water.

### 3-1-2. Production of HydroS

### Step (I) Dissolution and/or dispersion of low-solubility drugs in organic solvent

### Dissolution or dispersion in organic solvent

Low-solubility drugs can be dissolved in an organic solvent during heating and/or the application of pressure, in order to efficiently dissolve or disperse low-solubility drugs in an organic solvent.

### 3-2

### 3-2-1. Production of LipoS

### Step (II) Dissolving or dispersing lipophilic surfactant

When producing LipoS, lipophilic surfactant having an HLB value of 0 to less than 8 can be used. Examples of lipophilic surfactant include: sucrose fatty acid esters, such as sucrose stearic acid ester, sucrose palmitic acid ester, sucrose oleic acid ester, sucrose lauric acid ester, sucrose behenic acid ester, and sucrose erucic acid ester; sorbitan fatty acid esters, such as sorbitan monostearate, sorbitan tristearate, sorbitan monooleate, sorbitan trioleate, and sorbitan sesquioleate; fatty acid esters of glycerin, such as glycerol monostearate and glycerol monooleate; and polyglyceryl fatty acid esters, such as diglyceryl tetraisostearate, diglyceryl diisostearate, and diglyceryl monoisostearate. Such surfactant are dispersed or dissolved in an organic solvent. Two or more types of such lipophilic surfactant may be used, and a hydrophilic surfactant can be added in an amount that is half or less the amount of the lipophilic surfactant. Such amount is preferably 10% or less.

### 3-2-2. Production of HydroS

### Step (II) Dissolution or dispersion of hydrophilic surfactant

When producing HydroS, surfactant having an HLB value of 8 or greater can be used. Examples include anionic, cationic, amphoteric, and nonionic surfactant described below.

Examples of anionic surfactant that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, and sodium lauryl sulfate.

Examples of cationic surfactant that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactant that can be used include N-alkyl β-aminopropionic acid salt and N-alkyl β-iminodipropionic acid salt.

Examples of water-soluble nonionic surfactant that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among such nonionic surfactant, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, or polyglyceryl fatty acid ester can be preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil; trade name: Cremophor EL or EL-P, BASF Japan Ltd.) is particularly preferable. As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As polyoxyethylene polyoxypropylene glycol copolymers, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is preferable.

At least 1 type of such hydrophilic surfactant can be used, two or more types of such surfactant can be used, and a lipophilic surfactant can be added in an amount that is half or less the amount of the hydrophilic surfactant. Such amount is preferably 10% or less.

### 3-3. Step (III) Emulsification or dispersion

### 3-3-1. Production of LipoS

The aqueous solution comprising low-solubility drugs dissolved and/or dispersed therein obtained in step (I) (hereafter, it may be referred to as the "aqueous solution of step (I)") is mixed with the organic solvent comprising a lipophilic surfactant dissolved and/or dispersed therein obtained in step (II) (hereafter, it may be referred to as the "organic solvent of step (II)"), and the mixture is emulsified. The aqueous solution of step (I) may be added to the organic solvent of step (II), and the organic solvent of step (II) may be added to the aqueous solution of step (I). Preferably, the aqueous solution of step (I) and the organic solvent of step (II) are introduced into an emulsification tank while being heated and pressurized.

### 3-3-2. Production of HydroS

The organic solvent comprising low-solubility drugs dissolved and/or dispersed therein obtained in step (I) (hereafter, it may be referred to as the "organic solvent of step (I)") is mixed with an aqueous solution comprising a hydrophilic surfactant dissolved and/or dispersed therein obtained in step (II) (hereafter, it may be referred to as the "aqueous solution of step (II)"), and the mixture is emulsified. The organic solvent of step (I) may be added to the aqueous solution of step (II), and the aqueous solution of step (II) may be added to the organic solvent of step (I). Preferably, the organic solvent of step (I) and the aqueous solution of step (II) are introduced into an emulsification tank while being heated and pressurized.

When producing LipoS and HydroS, emulsification may be carried out with the use of, for example, a thin-film rotary high-speed agitator, a Hydromax mixer, an impeller agitator, or an Agi Homo mixer. That is, the air tightness of a shaft seal at the time of high-speed agitation while suppressing bumping with gas pressure is important, and a mechanical seal or the like is used.

### 3-4. Step (IV) Removal of solvent and/or moisture

When producing LipoS or HydroS, the step of removal of a solvent and/or moisture may be carried out in accordance with a conventional technique. Examples thereof include, but are not limited to, vacuum-freeze drying, drying under reduced pressure, and nitrogen purging. An optimal method of drying may be selected in accordance with the intended purpose.

The resulting S, a surfactant-drug composite, may be dispersed in water or a fat or oil by making use of the properties of surfactant. Also, such composite may be adsorbed to an adequate carrier such as silica and used for producing solid preparations, such as powders, granules, tablets, or capsules, as an active component.

### 4. Method for preparing S/O or S/O/W

Hereafter, a method for preparing S/O is described in detail.

S/O can be produced by the method comprising a step of dispersing LipoS produced in 3. above in at least 1 type of oil selected from the group consisting of a vegetable oil and a synthetic oil or fat.

S/O can be produced by the method comprising:
(A) a step of dissolving and/or dispersing low-solubility drugs in an aqueous solution to obtain a solution and/or dispersion;
(B) a step of liquefying a lipophilic surfactant via heating or other means or dissolving and/or dispersing a lipophilic surfactant in an organic solvent to obtain a solution and/or dispersion;
(C) a step of mixing the solution and/or dispersion obtained in step (A) with the solution and/or dispersion obtained in step (B) to obtain an emulsion or dispersion;
(D) a step of removing a solvent and/or moisture from the emulsion or dispersion obtained in step (C); and
(E) a step of dispersing the composite product of a low-solubility drug and surfactant obtained in step (D) in at least 1 type of oil selected from the group consisting of a vegetable oil and a synthetic oil or fat.

Also, S/O/W can be produced by a method comprising step (F) of dispersing S/O obtained in step (E) in purified water or regulated water, such as buffer.

(Step (A) and step (B) may be carried out in any order, and these steps may be carried out simultaneously. In the above-described method, further, low-solubility drugs may be added in the form of fine powder in step (B) to obtain a solution or dispersion. Alternatively, water may further be added thereto to obtain an emulsion.)

Hereafter, such steps are described in greater detail.

### 4-1. Step (A) Dissolution and/or dispersion of low-solubility drugs in aqueous solution

Low-solubility drugs can be dissolved in an aqueous solution with the use of a solubilizer. Examples of solubilizers for low-solubility drugs include water-soluble acids such as citric acid, adipic acid, lactic acid, phosphoric acid, and carbonic acid compounds, water-soluble bases such as sodium hydroxide, ammonium hydroxide, and sodium phosphate compounds, amino acids, proteins, urea, and clathrate compounds such as cyclodextrin, various types of nucleic acids, and various types of saccharides. Such solubilizers can be used alone or in combinations of two or more.

Heating and/or pressurization can also be carried out, in order to efficiently dissolve or disperse low-solubility drugs in an aqueous solution.

### 4-2. Step (B) Dissolution of lipophilic surfactant or dissolution or dispersion thereof in organic solvent

Lipophilic surfactant having an HLB value of 0 to less than 8 can be used. Examples of lipophilic surfactant include: sucrose fatty acid esters, such as sucrose stearic acid ester, sucrose palmitic acid ester, sucrose oleic acid ester, sucrose lauric acid ester, sucrose behenic acid ester, and sucrose erucic acid ester; sorbitan fatty acid esters, such as sorbitan monostearate, sorbitan tristearate, sorbitan monooleate, sorbitan trioleate, and sorbitan sesquioleate; fatty acid esters of glycerin, such as glycerol monostearate and glycerol monooleate; and polyglyceryl fatty acid esters, such as diglyceryl tetraisostearate, diglyceryl diisostearate, and diglyceryl monoisostearate. Such surfactant are dispersed or dissolved in an organic solvent. Two or more types of such lipophilic surfactant may be used, and a hydrophilic surfactant can be added in an amount that is half or less the amount of the lipophilic surfactant. Such amount is preferably 10% or less.

### 4-3. Step (C) Emulsification or dispersion

The aqueous solution comprising low-solubility drugs dissolved and/or dispersed therein obtained in step (A) (hereafter, it may be referred to as the "aqueous solution of step (A)") is mixed with the organic solvent comprising a lipophilic surfactant dissolved and/or dispersed therein obtained in step (B) (hereafter, it may be referred to as the "organic solvent of step (B)"), and the mixture is emulsified. The aqueous solution of step (A) may be added to the organic solvent of step (B), and the organic solvent of step (B) may be added to the aqueous solution of step (A). Preferably, the aqueous solution of step (A) and the organic solvent of step (B) are introduced into an emulsification tank while being heated and pressurized.

Emulsification may be carried out with the use of, for example, a thin-film rotary high-speed agitator, a Hydromax mixer, an impeller agitator, or an Agi Homo mixer. That is, the air tightness of a shaft seal at the time of high-speed agitation while suppressing bumping with gas pressure is important, and a mechanical seal or the like is used.

### 4-4. Step (D) Removal of solvent and/or moisture

The step of drying may be carried out in accordance with a conventional technique. Examples thereof include, but are not limited to, vacuum-freeze drying, drying under reduced pressure, and nitrogen purging. An optimal method of drying may be selected in accordance with the intended purpose.

### 4-5. Step (E) Preparation of S/O preparation

After the removal of moisture and/or a solvent, a composite of low-solubility drugs enclosed by surfactant and surfactant may be dispersed in an oil base to obtain an S/O preparation. Any oil components that can be used for pharmaceutical preparations can be used as an oil base without particular limitation. Examples thereof include a vegetable oil, an animal oil, a neutral lipid (monosubstituted, disubstituted, or trisubstituted glyceride), a synthetic oil or fat, and a sterol derivative.

Oil components are composed of at least one member selected from among a vegetable oil, a mineral oil, Vaseline, paraffin, oil or fats comprising saturated or unsaturated fatty acids, such as medium-chain saturated fatty acid, waxes, lanolin, fatty acid ester of glycerin, propylene glycol fatty acid ester, polyglyceryl fatty acid ester, sorbitan fatty acid ester, lecithin, acetyl glycerin fatty acid ester , polyethylene glycol, propylene glycol, polyoxyethylene polyoxypropylene glycol, triethyl citrate, triacetin, myristyl alcohol, cetanol, stearyl alcohol, glycerine, and ethanol.

Specific examples of vegetable oils include soybean oil, cottonseed oil, rapeseed oil, sesame oil, corn oil, peanut oil, safflower oil, sunflower oil, olive oil, and Perilla oil. Specific examples of animal oils include beef tallow, lard, and fish oil. Specific examples of neutral lipids include triolein, trilinolein, tripalmitin, tristearin, trimyristin, triarachidonin, squalane, and squalene. A specific example of a synthetic oil or fat is azone. Specific examples of sterol derivatives include cholesteryl oleate, cholesteryl linoleate, cholesteryl myristate, cholesteryl palmitate, and cholesteryl arachidate. These substances may be used in combinations of two or more.

Preferable examples of an oil component include triglyceride and a vegetable oil comprising, as an active component, the same.

Soybean oil is preferable in view of practical use. Highly purified soybean oil is particularly preferable.

The content of such oil component in the S/O suspension drug carrier of the present invention varies depending on the oil component type, other components, and other conditions. Such content is preferably from 50 to 99.5 W/V% and more preferably from 60 to 90 W/V%.

### 4-6. Step (F) Preparation of S/O/W preparation

S/O/W can be produced by dispersing S/O in purified water or regulated water, such as buffer. In the present invention, examples of water in which S/O comprises a composite of a low-solubility drug and surfactant dispersed in an oil base include water for injection and purified water that can be used for manufacturing pharmaceutical products, in accordance with the intended dosage forms, such as injection preparations, eye drops, oral preparations, and preparations for external use. Also, salts, saccharides, or water-soluble polymers may be added according to need.

Also, a hydrophilic surfactant may be added to the aqueous phase to prepare an S/O/W preparation. In such a case, a lipophilic or hydrophilic surfactant may be added to the S/O preparation in an amount that is half or less the amount of the hydrophilic surfactant that is added to the aqueous phase. Such amount is preferably 10% or less. Further, a lipophilic surfactant may be added to the aqueous phase. The amount of the lipophilic surfactant to be added to the aqueous phase is half or less the amount of the hydrophilic surfactant. Such amount is preferably 10% or less.

Examples of salts include: inorganic acids, such as hydrochloric acid and sulfuric acid, and various salts thereof; inorganic bases, such as caustic soda, potassium hydroxide, and calcium hydroxide, and various salts thereof; organic acids, such as succinic acid, citric acid, and stearic acid, and various salts thereof; and amino acids or organic bases and salts thereof. These substances may be used in combinations of two or more.

Further, examples of saccharides include: monosaccharides, such as glucose and galactose; oligosaccharides, such as lactose, sucrose, and trehalose; sugar alcohols, such as mannitol, sorbitol, and xylitol; and polysaccharides, such as hyaluronic acid, chondroitin sulfate, pullulan, pectin, hydroxypropyl methylcellulose, heparin, alginic acid, and carboxymethyl cellulose. These substances may be used in combinations of two or more.

Examples of water-soluble polymers include polyvinyl pyrrolidone, polyvinyl alcohol, albumin, and casein. These substances may be used in combinations of two or more.

### 5. S/W

Hereafter, a method for preparing S/W is described in detail.

S/W can be produced by the method comprising a step of dispersing HydroS produced in 3. above in purified water or an aqueous solution.

S/W can be produced by the method comprising:
(i) a step of dissolving and/or dispersing low-solubility drugs in an organic solvent to obtain a solution and/or dispersion;
(ii) a step of liquefying and dissolving a hydrophilic surfactant via heating or other means or dissolving and/or dispersing a hydrophilic surfactant in water to obtain a solution and/or dispersion;
(iii) a step of adding the solution and/or dispersion in an organic solvent obtained in step (i) to the solution and/or dispersion obtained in step (ii) to obtain an emulsion or dispersion;
(iv) a step of removing a solvent and/or moisture from the emulsion or dispersion obtained in step (iii) to obtain S; and
(v) a step of dispersing S obtained in step (iv) in purified water or regulated water, such as buffer. Step (i) and step (ii) may be carried out in any order, and these steps may be carried out simultaneously. In step (ii), further, low-solubility drugs may be added in the form of fine powder to obtain a solution or dispersion. Alternatively, an organic solvent may further be added thereto to obtain an emulsion.

Each step of the method for preparing S/W is described below in detail.

### 5-1. Step (i) Dissolution and/or dispersion of low-solubility drugs in organic solvent

Heating and/or pressurization can also be carried out, in order to efficiently dissolve and/or disperse low-solubility drugs in an organic solvent.

Examples of organic solvents include...

### 5-2. Step (ii) Dissolution (liquefaction) of hydrophilic surfactant or dissolving or dispersing thereof in water

When producing S/W, surfactant having an HLB value of 8 or greater can be preferably used. Examples include anionic, cationic, amphoteric, and nonionic surfactant described below.

Examples of anionic surfactant that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, and sodium lauryl sulfate.

Examples of cationic surfactant that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactant that can be used include N-alkyl β-aminopropionic acid salt and N-alkyl β-iminodipropionic acid salt.

Examples of water-soluble nonionic surfactant that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among nonionic surfactant, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, and polyglyceryl fatty acid ester can be preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil, trade name: Cremophor EL or EL-P, BASF Japan Ltd.) is particularly preferable. As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As polyoxyethylene polyoxypropylene glycol copolymers, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is particularly preferable.

Such hydrophilic surfactant can be used in combinations of two or more, and a lipophilic surfactant can be added in an amount that is half or less the amount of the hydrophilic surfactant. Such amount is preferably 10% or less.

### 5-3. Step (iii) Emulsification or dispersion

The aqueous solution comprising low-solubility drugs dissolved and/or dispersed therein obtained in step (i) (hereafter, it may be referred to as the "organic solvent of step (i)") is mixed with the aqueous solution comprising a hydrophilic surfactant dissolved and/or dispersed therein obtained in step (ii) (hereafter, it may be referred to as the "aqueous solution of step (ii)"), and the mixture is emulsified. The organic solvent of step (i) may be added to the aqueous solution of step (ii), and the aqueous solution of step (ii) may be added to the organic solvent of step (i). Preferably, the organic solvent of step (i) and the aqueous solution of step (ii) are introduced into an emulsification tank while being heated and pressurized.

Emulsification may be carried out with the use of, for example, a thin-film rotary high-speed agitator, a Hydromax mixer, an impeller agitator, or an Agi Homo mixer. That is, the air tightness of a shaft seal at the time of high-speed agitation while suppressing bumping with gas pressure is important, and a mechanical seal or the like is used.

### 5-4. Step (iv) Removal of solvent and/or moisture

The step of drying may be carried out in accordance with a conventional technique. Examples thereof include, but are not limited to, vacuum-freeze drying, drying under reduced pressure, and nitrogen purging. An optimal method of drying may be selected in accordance with the intended purpose.

### 5-5. Step (v) Preparation of S/W

After the removal of moisture and/or a solvent, a composite of low-solubility drugs enclosed by surfactant and surfactant may be dispersed in an aqueous solution or purified water to obtain an S/W preparation. As an aqueous solution or purified water, water for injection or purified water that can be used for manufacturing pharmaceutical products are used in accordance with the intended dosage forms, such as injection preparations, eye drops, oral preparations, and preparations for external use, which can be used as pharmaceutical preparations. Also, salts, saccharides, or water-soluble polymers may be added according to need.

Examples of salts include: inorganic acids, such as hydrochloric acid and sulfuric acid, and various salts thereof; inorganic bases, such as caustic soda, potassium hydroxide, and calcium hydroxide, and various salts thereof; organic acids, such as succinic acid, citric acid, and stearic acid, and various salts thereof; and amino acids or organic bases and salts thereof. These substances may be used in combinations of two or more.

Further, examples of saccharides include: monosaccharides, such as glucose and galactose; oligosaccharides, such as lactose, sucrose, and trehalose; sugar alcohols, such as mannitol, sorbitol, and xylitol; and polysaccharides, such as hyaluronic acid, chondroitin sulfate, pullulan, pectin, hydroxypropyl methylcellulose, heparin, alginic acid, and carboxymethyl cellulose. These substances may be used in combinations of two or more.

Examples of water-soluble polymers include polyvinyl pyrrolidone, polyvinyl alcohol, albumin, and casein. These substances may be used in combinations of two or more.

### 6. Apparatus for production

Fig. 4 shows an embodiment of an apparatus for production used in the process of production according to the present invention. This apparatus comprises a water tank, an organic solvent tank, and an emulsification tank. After a solution comprising a low-solubility drug or surfactant added thereto is added to each of such tanks, one or more types of air or nonflammable gas is introduced thereinto, and the liquid level is maintained in a pressurized state with such gas phase. Thus, water and an organic solvent having different boiling points can be emulsified with heating.

Such tanks are heated at an appropriate temperature in accordance with physical properties of low-solubility drugs to dissolve the contents. The resultants may be subjected to emulsification with the organic solvent, which has been similarly heated, by heating. Thus, very fine micelles can be prepared.

Emulsification may be carried out with the use of, for example, a thin-film rotary high-speed agitator, a Hydromax mixer, an impeller agitator, an Agi Homo mixer, a homogenizer, or an ultrasonic processor. That is, the air tightness of a shaft seal at the time of high-speed agitation while suppressing bumping with gas pressure is important, and a mechanical seal or the like is used. Further, a wet method is more preferable than a dry method, in order to prevent very fine powders, such as carbon or ceramic powders, from being diffused in the sample due to high-speed rotation. It should be noted that such method would not restrict utilization of other techniques.

When rip seals other than mechanical seals are used, seals are in contact with each other. This may cause contamination of the solution with seal material due to friction. When a high level load is applied, it is highly likely that foreign matter would be generated. When mechanical seals are used, seals are not in contact with each other (this necessitates the use of seal water), and the tank rotates at a high speed while seal water is sandwiched by seals. Thus, it is highly unlikely that foreign matter would be generated.

The step of emulsification may be carried out with the use of a nanomizer machine, which is a high-pressure emulsifier. Alternatively, an emulsifier involving the use of a liquid-phase laser ablation apparatus or ultrasonic waves may be used instead of mechanical agitation. As described above, formation of microemulsions during heating and pressurization is sufficient, and it would not restrict the use of other equipment.

Micelles (W/O), which had been completed the step of emulsification, become a composite of a low-solubility drug and surfactant (solid) after the step of removal of moisture and/or a solvent. The step of drying may be carried out in accordance with a conventional technique. Examples thereof include, but are not limited to, vacuum-freeze drying, drying under reduced pressure, and nitrogen purging. An optimal method of drying may be selected in accordance with the intended purpose.

### 7. Drug preparation

As components of a base material, soybean protein, silk protein, hyaluronic acid, a water-soluble polymer, such as hydroxypropyl cellulose, polyvinyl alcohol, hydroxypropyl methylcellulose, or polyvinyl pyrrolidone, or a lipophilic polymer such as polyethylene, casein, or silicic acid anhydride can be contained as a dispersion stabilizer. Also, a stabilizer, such as cetanol, myristyl alcohol, or stearyl alcohol, may be added, in order to enhance physical strength or storage stability.

A stabilizer, such as tocopherol, citric acid, phytic acid, sodium hydroxide, or monoethanolamine, can be contained, so as to prevent oxidation, degradation, or polymerization.

In the S/O suspension of the present invention, the content of the emulsifier varies depending on the type of emulsifier, other components, or other conditions. Such content is preferably from 0.05 to 50 W/V% and more preferably from 0.1 to 40 W/V%.

In accordance with the intended purpose of preparations, an S/O suspension comprising a composite of a low-solubility drug and surfactant (solid) dispersed in a fat or oil, an S/W suspension comprising such composite dispersed in purified water or water for injection that can be used for pharmaceutical products, or an S/O/W suspension comprising the S/O suspension dispersed in water (e.g., purified water) can be prepared.

The S/W suspension may be any solution, provided that the composite of a low-solubility drug and surfactant comprising low-solubility drugs enclosed by a hydrophilic surfactant can be used for producing pharmaceutical products. Examples thereof that can be used include pure water, purified water, physiological saline, and buffer.

Also, S/O/W is prepared by microdispersing the S/O suspension in purified water or water for injection. Salts, amino acids, saccharides, water-soluble polymers, or the like may be arbitrarily added to purified water or water for injection to suppress formation of an aggregate of micro-dispersed solids (S or S/O), unless otherwise specified. The amount thereof to be added varies depending on low-solubility drug content, and such amount can be selected in accordance with the intended purpose.

Examples of salts include sodium chloride, potassium chloride, calcium chloride, sodium borate, sodium phosphate, and sodium citrate. These substances may be used in combinations of two or more.

Various types of neutral, acidic, and basic amino acids can be used for adjusting pH levels. Examples thereof include L-glycine, L-proline, L-alanine, L-glutamic acid, L-aspartic acid, L-arginine, and L-lysine. These substances may be used in combinations of two or more.

Examples of saccharides include glucose, lactose, maltose, sucrose, trehalose, and cellobiose. These substances may be used in combinations of two or more.

Examples of water-soluble polymers include alginic acid, polyacrylic acid, polymethacrylic acid, carboxymethyl cellulose, hydroxypropyl methylcellulose, carragheenan, heparin, pullulan, and pectin. These substances may be used in combinations of two or more.

Examples of aqueous liquid preparations that can be used in the present invention include injection preparations, eye drops, aerosol, nasal drops, and enema suppositories.

Such aqueous liquid preparations can use various pH buffers, viscosity control agents, preservatives, antiseptic agents, stabilizers, and the like according to need.

Examples of dosage forms of preparations for internal use that are employed in the present invention include capsules, tablets, powders, granules, dusting powder, jelly formulations, and liquid preparations for internal use. Any dosage forms that are employed for preparations for internal use can be employed without particular limitation.

The preparations for internal use can further comprise a bitterness inhibitor, a coloring inhibitor, a stabilizer, a preservative, a binder, a fluidizer, and the like according to need.

Examples of excipients that are used for the preparations for internal use include: saccharides such as saccharose; starch derivatives such as dextrin; cellulose derivatives such as carmellose sodium; and water-soluble polymers such as xanthan gum.

Further, general amounts of coloring agents, lubricants (e.g., metallic stearates, such as calcium stearate and magnesium stearate; lauryl sulfate salts, such as sodium lauryl sulfate and magnesium lauryl sulfate; and starch derivatives used for the aforementioned excipients), binders, emulsifiers, thickeners, wetting agents, stabilizers (e.g., parahydroxybenzoates, such as methylparaben and propylparaben; alcohols, such as chlorobutanol, benzyl alcohol, and phenylethyl alcohol; benzalkonium chloride; phenols, such as phenol and cresol; thimerosal; acetic anhydride; and sorbic acid), preservatives, solvents (e.g., water, ethanol, and glycerine), solubilizers, suspending agent (e.g., carmellose sodium), buffers, pH regulators, and the like can be incorporated.

S, S/O, S/W, and S/O/W can be prepared in the form of solid preparations via various methods. Examples of such methods include a method involving mixing of a lipid that is referred to as a hard fat having a high melting point and a method involving adsorption to porous powders. Porous powders to which the drugs are to be adsorbed are selected from powders or porous carriers, such as organic matter, such as saccharides, sugar alcohols, celluloses, cellulose derivatives, and starch derivatives and inorganic substances, such as silica, light anhydrous silicic acid, Neusilin, and Aerosil. Further, binders, disintegrators, foaming agents, sweetening agents, aroma chemicals, coloring agents, and the like can be added.

Examples of disintegrators include starches, starch derivatives, cellulose derivatives such as hydroxypropyl cellulose and cross carmellose, crospovidone, and agar disintegrator.

Examples of binders include hydroxypropyl cellulose, polyvinyl alcohol, hydroxypropyl methyl cellulose, polyvinyl pyrrolidone, partially saponified polyvinyl alcohol, methyl cellulose, and pullulan.

Examples of sweetening agents include Aspartame^{®}, saccharin, and glycyrrhizin. Examples of aroma chemicals include lemon, orange, pineapple, mint, and menthol. Examples of coloring agents include yellow iron sesquioxide, iron sesquioxide, and tar coloring.

Examples of the preparations for external use of the present invention include ointments, lotions, aerosols, plasters, aqueous cataplasms, oil plasters, and tapes, and any dosage forms that are employed for preparations for external use can be employed without particular limitation.

Hereafter, Production Examples and Examples are described, although the technical scope of the present invention is not limited thereto.

### (Production Example 1)

### (Production of S/W ubiquinone solution)

SDS (120 g) was completely dissolved in 1200 g of purified water, and 120 g of n-hexane comprising 40 g of CoQ10 dissolved therein was added thereto during agitation at 1,500 rpm using a lamond stirrer. The resulting solution was subjected to membrane emulsification with the use of a 0.1-um lipophilic membrane (a Teflon membrane), and the solution was frozen at -45°C after emulsification, followed by lyophilization. The resulting ubiquinone-surfactant composite (5 g) was completely dissolved in 45 g of purified water to obtain an S/W ubiquinone solution.

### (Production Example 2)

### (Production of S/O aciclovir solution)

Sucrose fatty acid ester (ER-290, 60 g) was completely dissolved in 450 g of hexane, the solution was heated to 100°C, and the solution was agitated at 20,000 rpm using a homogenizer. The resulting solution was designated as an organic solvent layer. Separately, 3 g of acyclovir was dispersed in 300 g of purified water, and the solution was heated to 100°C while agitating the solution at 6,000 rpm to completely dissolve aciclovir therein. Thereafter, the acyclovir solution was pressurized to 0.25 MPaG with the use of pressure in the container and the container of the organic solvent layer, the solution was transported to the organic solvent layer with pressure, and emulsification was then carried out at 20,000 rpm for 5 minutes. Thereafter, the temperature in the container was cooled to 40°C, and the hexane layer was removed with the use of a defoaming device. After the solvent was removed, the resultant was frozen at -45°C and lyophilized, and the solvent was completely removed. Medium-chain triglyceride (49 g) was added relative to 1 g of the resulting aciclovir-surfactant composite to obtain an S/O aciclovir solution.

### (Control Example 1)

### (Production of S/O aciclovir solution via conventional technique)

The solubility of aciclovir in water was very poor at room temperature (25°C), and an aqueous solution could not be prepared. Thus, an S/O solution could not be obtained. Thus, an aciclovir dispersion was prepared in the form of an S/O solution.

Sucrose fatty acid ester (ER-290, 60 g) was completely dissolved in 450 g of hexane, and the solution was agitated at 20,000 rpm. The resulting solution was designated as an organic solvent layer. Separately, 3 g of acyclovir was homogeneously dispersed in 300 g of purified water, the resulting solution was introduced into the organic solvent layer, and emulsification was then carried out at 20,000 rpm for 5 minutes. Thereafter, the hexane layer was removed with the use of a defoaming device. After the solvent was removed, the resultant was frozen at -45°C and lyophilized, and the solvent was completely removed. Medium-chain triglyceride (49 g) was added relative to 1 g of the resulting aciclovir-surfactant composite to obtain an S/O aciclovir solution.

### (Production Example 3)

### (Production of S/O diclofenac sodium solution)

Sucrose fatty acid ester (ER-290, 300 g) was completely dissolved in 450 g of hexane, the solution was heated to 100°C, the solution was agitated at 20,000 rpm, and the pressure in the container was raised to 0.10 MPaG with the use of a nitrogen gas. The resulting solution was designated as an organic solvent layer. Separately, 30 g of diclofenac sodium was dispersed in 300 g of purified water, the solution was heated to 65°C while agitating the solution at 6,000 rpm to completely dissolve diclofenac sodium therein, and the pressure in the container was raised to 0.10 MPaG with the use of a nitrogen gas. The resulting diclofenac sodium solution was transported to the organic solvent layer, and emulsification was carried out at 20,000 rpm for 5 minutes at a pressure of 0.1 MPaG. Thereafter, a temperature in the container was cooled to 40°C, and the n-hexane layer was removed with the use of a defoaming device. After the solvent was removed, the resultant was frozen at -45°C and lyophilized, and the solvent was completely removed. The resulting diclofenac sodium-surfactant composite (11 g) was diluted with medium-chain triglyceride to obtain 100 ml of a solution, and an S/O diclofenac sodium solution was thus obtained.

### (Control Example 2)

### (Production of S/O diclofenac sodium solution via conventional technique)

A dispersion of 10% diclofenac sodium (1 ml) was introduced into 20 g of the n-hexane solution comprising 5% by weight of sucrose erucic acid ester (ER290) dissolved therein accommodated in the emulsification tank, and the dispersion was subjected to high-speed agitation (20,000 rpm) with the use of a homogenizer to prepare a W/O emulsion solution.

The resulting emulsion solution was dried under reduced pressure, the solvent was removed therefrom, and the resultant was subjected to lyophilization overnight. Thus, the surfactant-diclofenac sodium composite (solid) was obtained. Medium-chain triglyceride (10 ml) was added to the resulting composite, and ultrasonic waves were applied thereto to obtain a diclofenac sodium dispersion.

### (Control Example 3)

### (Method for preparing S/O preparation according to conventional technique)

A solution of 1% diclofenac sodium (1 ml) was introduced into 20 g of the n-hexane solution comprising 5% by weight of sucrose erucic acid ester (ER290) dissolved therein accommodated in the emulsification tank, and the solution was subjected to high-speed agitation (20,000 rpm) with the use of a homogenizer to prepare a W/O emulsion solution. The resulting emulsion solution was dried under reduced pressure, the solvent was removed therefrom, and the resultant was subjected to lyophilization overnight. Thus, the surfactant-diclofenac sodium composite (solid) was obtained. Medium-chain triglyceride (10 ml) was added to the resulting composite, and ultrasonic waves were applied thereto to obtain an S/O diclofenac sodium solution.

### (Preparation Example 1)

### (Oral preparation of S/W ubiquinone)

Purified water (386 g) was introduced into a vacuum emulsifier of a I-litter vessel, 1 g of citric acid was added thereto, and the mixture was agitated to completely dissolve the content therein. Sorbitol (180 g) and a ubiquinone-surfactant composite (225 g) containing 4% (w/w) of ubiquinone that was prepared in the same manner as in Production Example 1 were introduced, the resultant was agitated at 1,500 rpm for 5 minutes, and an adequate amount of sodium citrate was added to adjust the pH level to 7.0. After the pH level was adjusted, the tank was heated to 60°C during agitation at 1,500 rpm. Separately, 5.4 g of carragheenan, 1.8 g of xanthan gum, and 0.9 g of purified locust bean gum were homogeneously dispersed in 90 g of concentrated glycerine. After the tank was heated to 60°C, a dispersion of concentrated glycerine-polysaccharide was slowly introduced to avoid lumps. After the whole amount was introduced, the agitation speed was raised to 5,000 rpm, and the temperature of the tank was raised to 85°C. Thereafter, the agitation speed was set at 3,000 rpm, and 0.9 g of propylparaben was introduced, followed by heating and sterilization for 30 minutes. The temperature was cooled to 60°C while maintaining the agitation speed at 3,000 rpm to obtain an S/W ubiquinone jelly preparation.

### (Preparation Example 2)

### (S/W ubiquinone preparation for external use)

Purified water (520 g) was introduced into a vacuum emulsifier of a I-litter vessel, 36 g of concentrated glycerine and 180 g of 4% carboxyvinyl polymer were added thereto, the mixture was agitated at 3600 rpm to homogeneously disperse the content therein, and the tank was heated to 80°C.

Stearic acid (10 g) and 90 g of squalane were introduced into another vessel, and the vessel was heated to 80°C to dissolve the content therein.

The squalane solution was introduced into the vessel, agitated at 3600 rpm for 5 minutes, and cooled to 60°C. Subsequently, 67.5 g of the ubiquinone-surfactant composite prepared in the same manner as in Production Example 1 and an adequate amount of paraben were introduced, and the mixture was agitated at 5400 rpm for 10 minutes *in vacuo* to obtain an S/W ubiquinone preparation for external use.

### Example 1

### (Solubility of S/W ubiquinone in water)

S/W ubiquinone was fractionated in amounts of 1 g, 2 g, 3 g, 4 g, and 5 g, and purified water was added thereto in order to adjust the total amount to 10 ml. The resultants were irradiated with ultrasonic waves for 1 hour, 3 ml of a solution portion was separated and filtered through a 0.22-µm mesh, and concentration was determined via HPLC using a catalyst column. As a result of analysis, the solutions were found to remain clear with S/W ubiquinone content of up to 4 g. S/W ubiquinone concentration was 24.7 mg/ml with S/W ubiquinone content of 1 g, 50.3 mg/ml with S/W ubiquinone content of 2 g, 76.2 mg/ml with S/W ubiquinone content of 3 g, and 102 mg/ml with S/W ubiquinone content of 4 g. The solution was somewhat cloudy with S/W ubiquinone content of 5 g, and S/W ubiquinone concentration was 109 mg/ml.

### Example 2

### Quantification of S/O aciclovir content (Production Example 2 and Control Example 1)

S/O acyclovir (2.1 g) was dispersed in 10 ml of 0.1N sodium hydroxide, and 5 ml of propylene glycol was added thereto to prepare a homogeneous solution. The amount of the solution was brought to 100 ml with the mobile phase for HPLC and the solution was analyzed via HPLC. As a result of analysis, concentration was found to be 14.2 mg/ml, which was consistent with concentration of the measured S/O aciclovir fraction.

### Example 3

### Particle size distribution of S/O acyclovir of Preparation Example 2

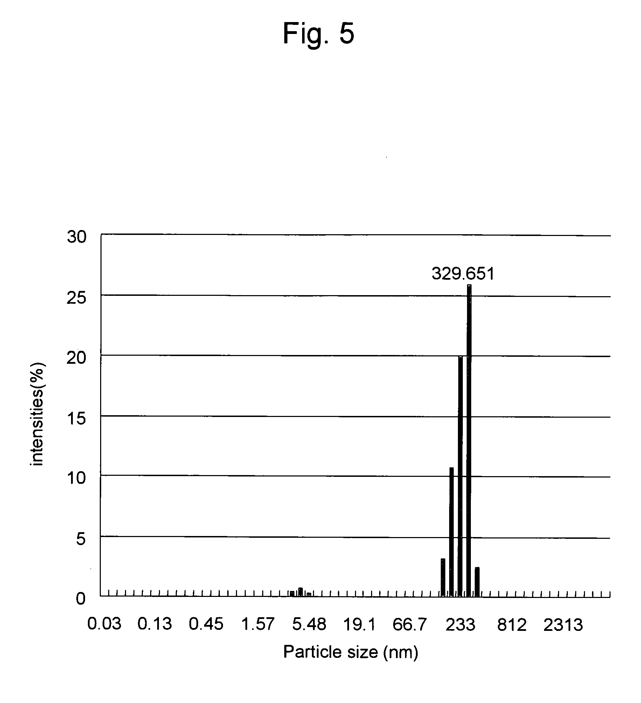

The sample of Production Example 2 was diluted 10-fold with hexane and assayed with the use of a particle size analyzer (SALD-3100, Shimadzu Co., Ltd.). The results are shown in Fig. 5.

### Example 4

### Micelle formation (Production Example 2) and crystal deposition (Control Example 1)

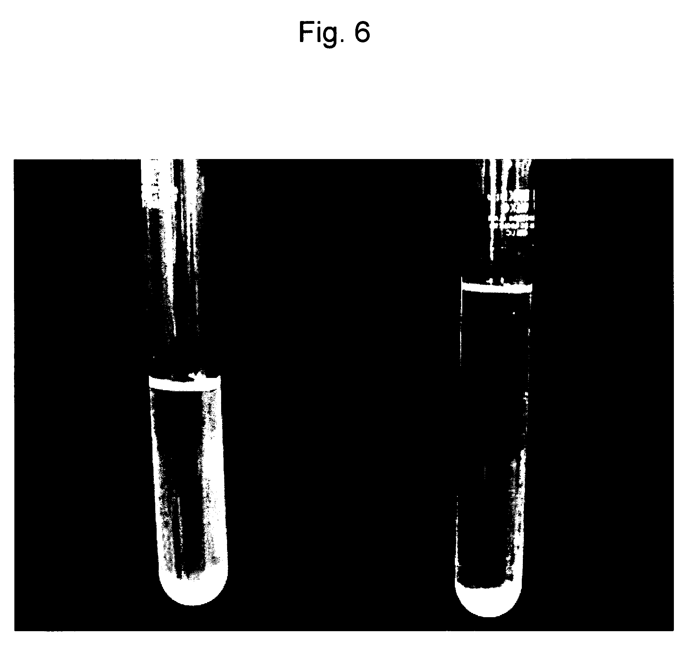

The results are shown in Fig. 6.

The left column represents Control Example 1 and the right column represents Production Example 2. While the sample of Production Example 2 maintains a condition in which micelles are homogeneously dispersed in the solution, crystals are deposited and aciclovir is precipitated in the sample of Control Example 1.

### Example 5

### Test of diclofenac sodium content (Production Example 3 and Control Example 2)

While the preparation prepared in Production Example 3 had the appearance of a clear solution, that of the preparation prepared in Control Example 2 was a white suspension.

### Example 6

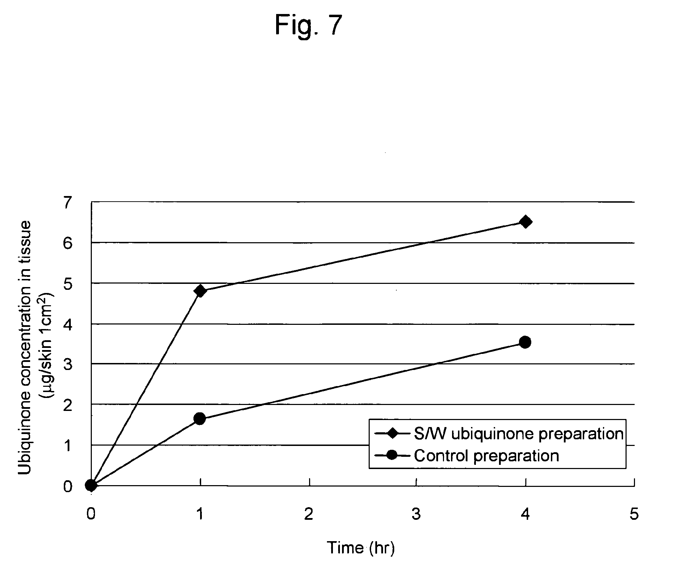

Hair in the abdominal region of an S. D. rat was removed by applying a depilatory cream on the day before the experiment, 50 µl of the 2% S/W ubiquinone preparation produced in Production Example 1 was applied to a 1-cm² region on the abdominal skin, and ubiquinone concentration in the tissue was measured 1 hour and 4 hours after application. As a control sample, a solution of 2% ubiquinone in ethanol was administered to a rat in the same manner. The results are shown in Fig. 7. The amount of the S/W ubiquinone preparation absorbed was 3 times higher than that of the control preparation 1 hour later, and it was 1.8 times higher 4 hours later.

### Example 7

### Percutaneous administration of S/O diclofenac sodium to rabbit's auricle (Production Example 3 and Control Example 3)

The drug was applied in an amount of 5 mg/kg to the left ear auricle, about 2 ml of blood was sampled 1, 2, 4, 6, 8, and 24 hours later from the external ear vein of the other ear; i.e., the right ear, the sampled blood was subjected to extraction in accordance with a general procedure, and the blood level was quantified via HPLC to determine AUC.

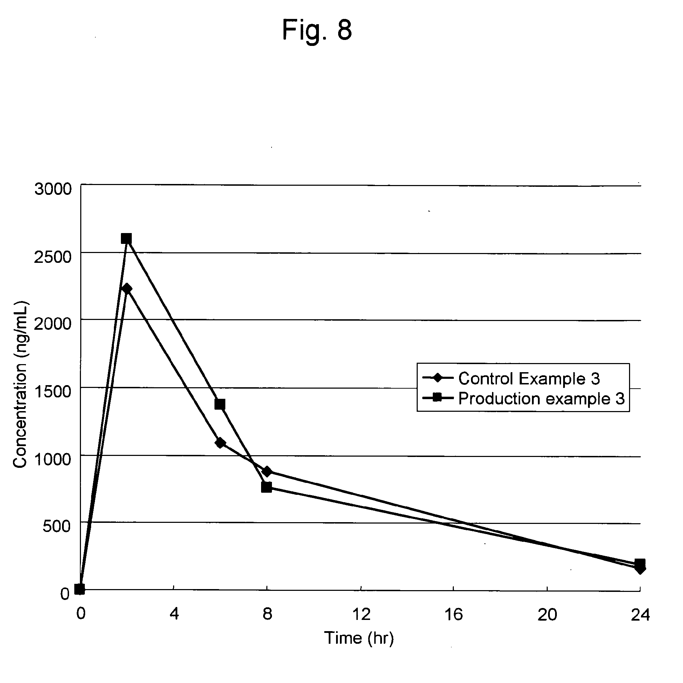

The results are shown in Fig. 8.

Regarding AUC of S/O diclofenac sodium, S/O of Production Example 3 exhibits 17.2 µg·h/ml and S/O of Control Example 3 exhibits 18 µg·h/ml; that is, such values are substantially equal to each other. Accordingly, preparation of S/O according to the method of the present invention yields an extent of bioavailability equivalent to that of the S/O preparation produced by a conventional method for S/O preparation. Since S/O was prepared at high temperature and high pressure in Production Example 3, the concentration of the resulting solution can be 10 times higher than that attained by the method for preparing S/O of the Control Examples.

### Industrial Applicability

In the present invention, low-solubility drugs classified as Class 2 or 4 according to BCS were enclosed by highly lipophilic surfactant, the resultant was dispersed in an oil to obtain an S/O dispersion, and the resultant was further dispersed in water to obtain an S/O/W dispersion. Alternatively, a complosite of low-solubility drugs enclosed by highly hydrophilic surfactant was prepared, and the resultant was dispersed in water to obtain an S/W dispersion. Thus, the present invention succeeded in modifying such low-solubility drugs into those of Class 1, which are excellent in solubility (HS) and permeability (HP).

According to the method for preparing a composite of a low-solubility drug and surfactant of the present invention, further, when dispersing drugs or surfactant or mixing the aqueous phase, which was melted via heating, with the organic solvent phase, air or nonflammable gas is charged so as to maintain a pressure of 1 to 10 atm at the gas phase in the upper portion of the liquid level. Thus, bumping or explosion caused at the time of emulsification with heating can be inhibited, and formation of fine W/O or O/W micelles can be realized via adequate selection of a solvent in accordance with physical properties of drugs.

By completely removing moisture or an organic solvent, a composite of a low-solubility drug and surfactant can be prepared, and S/W, S/O, or S/O/W can be prepared based thereon. Thus, the present invention can provide a drug having physical properties that are excellent in solubility and permeability. Further, S/W, S/O, or S/O/W comprising such composite of a low-solubility drug and surfactant can be used for food or cosmetic products. Also, pharmaceutical preparations comprising two or more types of water-soluble drugs dispersed in water or in a medium in an oil or fat can be prepared.

The present invention relates to a pharmaceutical preparation that is excellent in solubility and permeability, which is obtained by enclosing low-solubility drugs classified as Class 2 or 4 according to BCS with highly hydrophilic surfactant to obtain a composite of an S/W preparation or enclosing such low-solubility drugs with highly lipophilic surfactant to prepare a composite, dispersing the resulting composite in an oil to obtain an S/O dispersion, and dispersing the resultant in purified water or the like to obtain S/O/W preparations. The present invention also relates to production of the same.

All publications, patents, and patent applications cited herein are incorporated herein by reference in their entirety.

## Claims

1. A method for preparing a composite of a low-solubility drug and surfactant (hereafter, it may be referred to as "solid" or "S") comprising:
(1) a step of emulsifying and/or dispersing low-solubility drugs in a solvent using a surfactant; and
(2) a step of removing a solvent and/or moisture from the resulting emulsion and/or dispersion.

2. A method for preparing an S/O (Solid-in-Oil) or S/O/W (Solid-in-Oil-in-Water) preparation comprising:
(1) a step of emulsifying and/or dispersing low-solubility drugs in a solvent using a surfactant;
(2) a step of removing a solvent and/or moisture from the resulting emulsion and/or dispersion to obtain a composite product of a low-solubility drug and surfactant; and
(3) a step of dispersing the composite product of a low-solubility drug and surfactant in at least 1 type of oil selected from the group consisting of a vegetable oil and a synthetic oil or fat.

3. The method according to claim 2, wherein the step of emulsifying or dispersing low-solubility drugs in a solvent using a surfactant comprises:
(A) a step of dissolving or dispersing low-solubility drugs in an aqueous solution to obtain a solution and/or dispersion;
(B) a step of liquefying and dissolving a lipophilic surfactant via heating or dissolving and/or dispersing a lipophilic surfactant in an organic solvent to obtain a solution and/or dispersion; and
(C) a step of mixing the solution and/or dispersion obtained in step (A) with the solution and/or dispersion obtained in step (B) to obtain an emulsion or dispersion.

4. The method according to claim 3, wherein the step of emulsifying and/or dispersing low-solubility drugs in a solvent using a surfactant further comprises a step of dissolving or dispersing low-solubility drugs in the form of fine powder in step (B) or further adding water thereto to obtain an emulsion.

5. The method for preparing S/O/W according to any one of claims 2 to 4, which further comprises a step of dispersing the prepared S/O in purified water or regulated water, such as buffer.

6. The method according to any one of claims 2 to 5, wherein a lipophilic surfactant having an HLB value of less than 8 is used.

7. The method according to claim 6, wherein a hydrophilic surfactant having an HLB value of 8 or greater is added to the lipophilic surfactant having an HLB value of less than 8 in an amount that is half or less the amount of the lipophilic surfactant.

8. A method for preparing an S/W (Solid-in-Water) preparation comprising:
(1) a step of emulsifying and/or dispersing low-solubility drugs in a solvent using a surfactant;
(2) a step of removing a solvent and/or moisture from the resulting emulsion and/or dispersion to obtain a composite product of a low-solubility drug and surfactant (S); and
(3) a step of dispersing the composite product of a low-solubility drug and surfactant in purified water or buffer (regulated water).

9. The method for preparing S/W according to claim 8, wherein the step of emulsifying and/or dispersing low-solubility drugs in a solvent using surfactant comprises:
(i) a step of dissolving or dispersing low-solubility drugs in an organic solvent to obtain a solution and/or dispersion;
(ii) a step of liquefying and dissolving a hydrophilic surfactant via heating or dissolving and/or dispersing a hydrophilic surfactant in water to obtain a solution and/or dispersion; and
(iii) a step of mixing the solution and/or dispersion in an organic solvent obtained in step (i) with the solution and/or dispersion obtained in step (ii) to obtain an emulsion.

10. The method according to claim 9, wherein low-solubility drugs are added in the form of fine powder in step (ii) or an organic solvent is further added to thereto to obtain an emulsion.

11. The method according to any one of claims 8 to 10, wherein a hydrophilic surfactant having an HLB value of greater than 8 is used.

12. The method according to claim 11, wherein a lipophilic surfactant is added in an amount that is half or less the amount of the hydrophilic surfactant.

13. The method according to any one of claims 1 to 12, wherein dispersion, dissolution, and/or emulsification in the above steps are carried out by introducing air or nonflammable gas into the gas phase in the upper portion of the liquid level at a pressure of 1 to 10 atm.

14. The method according to claim 13, wherein the nonflammable gas is at least one type of gas selected from the group consisting of nitrogen, carbonic acid, helium, and argon gases.

15. The method according to any one of claims 1 to 14, wherein removal of a solvent and/or moisture is carried out by means of vacuum-freeze drying, drying under reduced pressure, microwave drying by bulking method, freeze-drying and grinding, or spray drying.

16. The method according to any one of claims 1 to 15, wherein the low-solubility drugs exhibit a degree of solubility that is equal to or less than the maximal drug content in 1 unit of the relevant preparation in 250 ml of buffer with a pH level of 1.0 to 7.5.

17. A composite product of a low-solubility drug and surfactant comprising low-solubility drugs enclosed by surfactant prepared by the method according to claim 1.

18. A product comprising the composite of a low-solubility drug and surfactant, which is the S/O (Solid-in-Oil) preparation prepared by the method according to any one of claims 2 to 7 and 13 to 16.

19. A product comprising the composite of a low-solubility drug and surfactant, which is the S/O/W (Solid-in-Oil-in-Water) preparation comprising the S/O (Solid-in-Oil) product comprising the composite of a low-solubility drug and surfactant according to claim 18 dispersed in an aqueous solution.

20. A product comprising the composite of a low-solubility drug and surfactant, which is the S/W (Solid-in-Water) preparation prepared by the method according to any one of claims 8 to 16.

21. A composite product of a low-solubility drug and surfactant comprising low-solubility drugs enclosed by surfactant, wherein the low-solubility drugs are enclosed in a solid state (hereafter, it is abbreviated as "Solid" or "S").

22. The composite product of a low-solubility drug and surfactant according to claim 21, wherein the low-solubility drugs exhibit a degree of solubility that is equal to or less than the maximal drug content in 1 unit of the relevant preparation in 250 ml of buffer with a pH level of 1.0 to 7.5.

23. A product comprising the composite of a low-solubility drug and surfactant, which is the S/W (Solid-in-Water) preparation comprising the composite of a low-solubility drug and surfactant according to claim 21 or 22 dispersed in water.

24. A product comprising the composite of a low-solubility drug and surfactant, which is the S/O (Solid-in-Oil) preparation comprising the composite of a low-solubility drug and surfactant according to claim 21 or 22 dispersed in oil.

25. A product comprising the composite of a low-solubility drug and surfactant, which is the S/O/W (Solid-in-Oil-in-Water) preparation comprising the S/O (Solid-in-Oil) product comprising the composite of a low-solubility drug and surfactant according to claim 24 dispersed in water.

26. The product comprising the composite of a low-solubility drug and surfactant according to any one of claims 18 to 25, wherein the low-solubility drugs are classified as Class 2 or 4 in accordance with the Biopharmaceutical classification system (BCS).
